# EUROPÄISCHE PATENTSCHRIFT

(11) **EP 0 641 571 B1**
(45) Veröffentlichungstag und Bekanntmachung des Hinweises auf die Patenterteilung: **17.10.2001**
(21) Anmeldenummer: 94110717.9
(22) Anmeldetag: 09.07.1994
(51) Int. Cl.: A61M 25/06

(54) **Venenpunktionskanüle für die Katheterverlegung**
Vein puncture cannula for catheter placement
Canule de ponction d'une veine pour la mise en place d'un cathéter

(30) Priorität: 08.09.1993 DE 4330400
(43) Veröffentlichungstag der Anmeldung: 08.03.1995
(73) Patentinhaber: B. BRAUN MELSUNGEN AG, 34212 Melsungen (DE)
(72) Erfinder: Raines, Kenneth C., Bethlehem, PA 18018 (US); McGaughey, John, Bethlehem, PA 18018 (US); Peppel, Peter, Bethlehem, PA 18018 (US)
(74) Vertreter: Selting, Günther, Dipl.-Ing.

(56) Entgegenhaltungen:
- DE-A- 3 100 545
- DE-A- 4 136 051
- US-A- 5 147 314
- US-A- 5 195 980

## Beschreibung

Die Erfindung betrifft eine Venenpunktionskanüle für die Katheterverlegung nach der Seldinger-Technik, mit einer Punktionsnadel, die ein Y-förmiges Ansatzstück mit zwei Ansätzen aufweist, wobei ein Ansatz zum Einschieben eines Führungsdrahtes und ein anderer Ansatz für den Anschluß einer Spritze dient.

Zum Einführen eines Katheters in eine Vene wird häufig die Seldinger-Technik angewandt. Hierbei erfolgt zunächst eine Punktion der Vene mit einer Punktionskanüle, an die eine Spritze angeschlossen ist. Der Punktionserfolg kann dadurch festgestellt werden, daß beim Aspirieren Blut durch die Punktionskanüle in die Spritze eindringt. Dieses Blut ist durch die durchsichtige Wand des Spritzenzylinders sichtbar. Dann wird die Spritze von dem Ansatzstück der Punktionskanüle entfernt und ein Führungsdraht durch die Punktionskanüle hindurch in die Vene eingeführt. Die Punktionskanüle wird über den Führungsdraht hinweg zurückgezogen und anschließend wird über den Führungsdraht ein Katheter vorgeschoben. Schließlich wird der Führungsdraht aus dem Katheter herausgezogen und das proximale Katheterende wird auf der Haut des Patienten fixiert. Eine Schwierigkeit bei der Seldinger-Technik besteht darin, daß nach der Punktion und vor dem Einschieben des Führungsdrahtes in das Ansatzstück bei positivem Venendruck Blut aus dem Ansatzstück herausfließt. Das herausfließende Blut verschmutzt die Umgebung und kann darüberhinaus eine Infektionsgefahr für den Arzt hervorrufen. Bei negativem Venendruck besteht die Gefahr einer Luftembolie.

Eine bekannte Venenpunktionskanüle nach DE 41 36 051 A1 weist am rückwärtigen Ende einer hohlen Punktionsnadel ein Ansatzstück auf, das in geradliniger Verlängerung der Punktionsnadel einen Ansatz hat, durch den der Führungsdraht hindurchgeschoben werden kann. Dieser Ansatz enthält eine Dichtung, die von dem Führungsdraht durchstoßen werden kann. Ferner weist das Ansatzstück einen seitlich rechtwinklig abzweigenden und nach hinten umgebogenen Ansatz auf, der zum Anschließen einer Spritze bestimmt ist, wobei die Spritze im wesentlichen parallel zu dem Drahtführungsansatz verläuft. Bei der Punktion des Blutgefäßes ist die Spritze zwar parallel zur Punktionsnadel ausgerichtet, jedoch seitlich versetzt. Daher müssen die Finger des Arztes beim Punktieren das Ansatzstück halten, führen und vorschieben.

Aus US 3 920 013 ist eine Punktionsvorrichtung bekannt, die eine Y-förmige Punktionsnadel aufweist. An einem geradlinig durchgehenden Schenkel der Punktionsnadel wird die Spritze befestigt, die bei der Punktion zugleich als Handgriff für die Punktionsnadel dient. Ein von der Punktionsnadel abzweigender Schenkel dient als Einführkanal für einen weichen Katheter, der durch die Punktionskanüle hindurch vorgeschoben werden kann. Wenn der Katheter auf diese Weise verlegt ist, wird die Punktionskanüle zurückgezogen, bleibt aber über dem Katheter, von dem sie nicht nach hinten abgezogen werden kann.

Ferner sind Katheter-Einführungsvorrichtungen mit selbstschließenden Ventilen, die den Durchlaß eines Katheters oder auch eines Führungsdrahtes ermöglichen, bekannt aus US 5 195 980 und US 5 102 395. Eine solche Führungsvorrichtung, die als Y-Stück ausgebildet sein kann, hat ein selbstschließendes Ventil in dem geradlinig durchgehenden Katheterkanal. Diese Führungsvorrichtungen sind nicht als Punktionskanülen geeignet, die sowohl die Punktion eines Blutgefäßes ermöglichen als auch das Einschieben eines Führungsdrahtes in das Blutgefäß.

Eine Venenpunktionskanüle, von der der Oberbegriff des Patentanspruchs 1 ausgeht, ist bekannt aus US-A-5 147 314. Diese Venenpunktionskanüle weist eine Punktionsnadel mit einem Y-förmigen Ansatzstück auf. Der gerade durchgehende erste Ansatz dient zum Anbringen einer Spritze, während der schräg nach hinten abstehende zweite Ansatz dazu benutzt wird, einen Führungsdraht in die Punktionsnadel einzuschieben. Der zweite Ansatz enthält einen geschlossenen durchstechbaren Stopfen. Der Führungsdraht ist in einem Adapter enthalten, welcher eine Hohlnadel zum Durchstechen des Stopfens des Y-Ansatzstückes aufweist. Durch diese Hohlnadel hindurch wird der Führungsdraht in die Punktionsnadel eingeschoben.

US-A-5 195 980 beschreibt ein Y-förmiges Ansatzstück, dessen geradlinig durchgehender erster Ansatz eine selbstschließende Ventilvorrichtung enthält. Diese Ventilvorrichtung besteht aus einem Schlitzventil und einer elastischen Lochscheibe. Sie wird durch eine Nadel geöffnet, die Bestandteil eines federgespannten Betätigungsstückes ist. Durch die hohle Nadel wird ein auf einem Führungsdraht sitzender Katheter hindurchgeschoben. Anschließend wird die hohle Nadel zurückgezogen, so daß sich die Ventilvorrichtung um den Katheter herum schließt.

Der Erfindung liegt die Aufgabe zugrunde, eine Venenpunktionskanüle der im Oberbegriff des Patentanspruchs 1 angegebenen Art zu schaffen, bei der die Handhabung, insbesondere während der Punktion, erleichtert ist.

Die Lösung dieser Aufgabe erfolgt erfindungsgemäß mit den Merkmalen des Patentanspruchs 1.

Bei der erfindungsgemäßen Venenpunktionskanüle bildet der koaxial zur Stahlnadel verlaufende erste Ansatz den Spritzenhalter und der hiervon abzweigende zweite Ansatz enthält die Ventilvorrichtung. Dadurch, daß der koaxiale erste Ansatz die Spritze trägt, kann der Spritzenzylinder als Handgriff zum Vorschieben der Punktionskanüle während der Punktion benutzt werden. Der Spritzenzylinder ist dabei koaxial zur Punktionsnadel ausgerichtet, so daß er eine exakte und feinfühlige Führung der Punktionskanüle ermöglicht. Der Führungsdraht wird durch den zweiten Ansatz hindurch unter spitzem Winkel in das Lumen der Punktionsnadel eingeführt. Obwohl ein Führungsdraht für die Seldinger-Technik eine relativ große Steifigkeit hat, die viel größer ist als die Steifigkeit eines Katheters, hat sich ergeben, daß ein solcher Führungsdraht die Abbiegung durch den abzweigenden Ansatz eines Y-Stückes mitmacht und durch diesen Ansatz auch herausgezogen werden kann, ohne daß übermäßige Reibung oder die Gefahr einer unbeabsichtigten Verschiebung der Venenpunktionskanüle eintritt.

Die erfindungsgemäße Venenpunktionskanüle eignet sich insbesondere für die supraclaviculäre Anonymapunktion zur Katheterisierung der Vena anonyma mit der Seldinger-Technik. Die Vena anonyma sinistra und die Vena anonyma dextra liegen im Halsbereich und vereinigen sich zur Vena cava superior. Die Vena anonyma stellt auch bei schwerem Schock einen sicheren venösen Zugang dar. Das Punktionsbesteck ist u.a. auch für die Punktion der Vena jugularis oder der internen und externen Vena subclavia geeignet.

Im folgenden wird unter Bezugnahme auf die Zeichnungen ein Ausführungsbeispiel der Erfindung näher erläutert.

Es zeigen:
- Fig. 1: eine Ansicht der Venenpunktionskanüle mit Spritze und Führungsdraht,
- Fig. 2: in vergrößertem Maßstab eine Darstellung der Einzelheit II aus Fig. 1,
- Fig. 3: eine Explosionsdarstellung der Teile des Ansatzstückes,
- Fig. 4: einen Längsschnitt durch das Ansatzstück und
- Fig. 5: eine perspektivische Ansicht des Schlitzventils.

Die Katheter-Einführungsvorrichtung bildet ein Besteck, das aus folgenden Teilen besteht: der Punktionskanüle mit Punktionsnadel 10 und Ansatzstück 11, der Spritze 12 und dem flexiblen Führungsdraht 13. Der Katheter, der nach Entfernen der Punktionskanüle über den Führungsdraht 13 geschoben wird, ist nicht dargestellt.

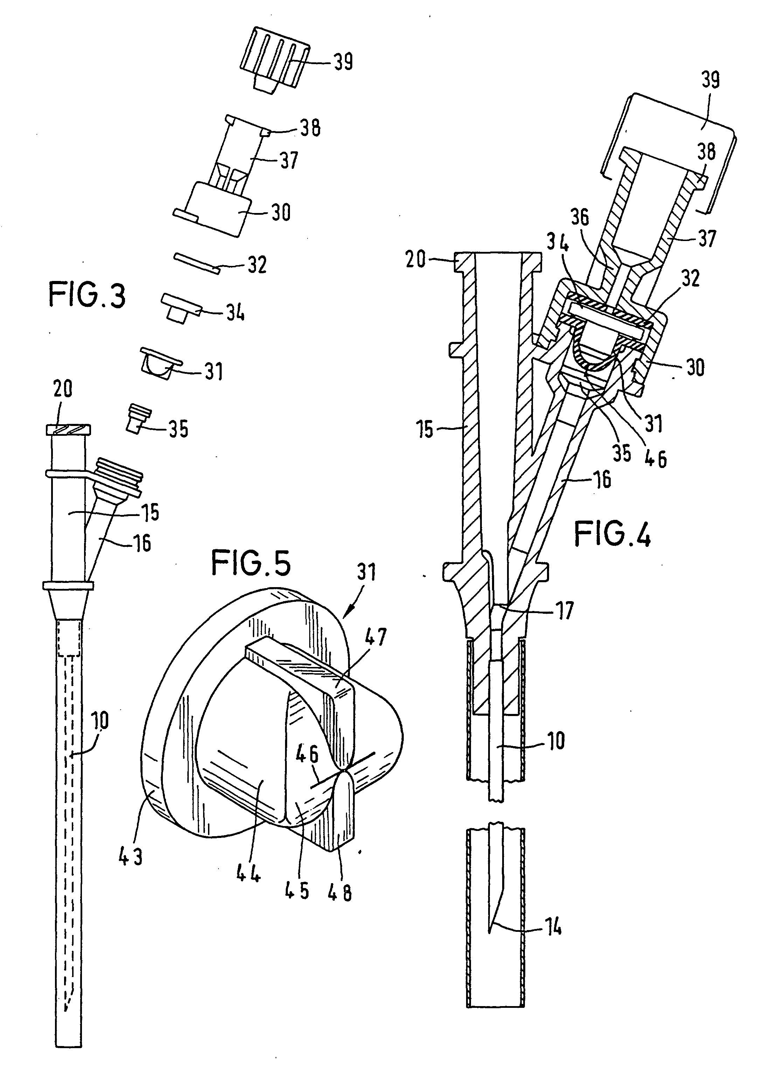

Die Punktionskanüle weist eine hohle Punktionsnadel aus Stahl auf, die am distalen (vorderen) Ende eine scharfe Spitze 14 aufweist. Am proximalen Ende der Stahlnadel 10 ist das aus Kunststoff bestehende Y-förmige Ansatzstück 11 befestigt. Dieses Ansatzstück weist einen ersten Ansatz 15 auf, der in Längsrichtung der Punktionsnadel 10 ausgerichtet ist, und einen zweiten Ansatz 16, der schräg unter einem Winkel von weniger als 35°, hier etwa 30°, von dem ersten Ansatz 15 abgeht. Jeder der Ansätze 15 und 16 enthält einen längslaufenden Kanal. Beide Kanäle vereinigen sich im Ansatzstück 11 an einer Übergangsstelle 17 (Fig. 4), die mit dem Lumen der Punktionsnadel 10 in Verbindung steht. Die Punktionsnadel 10 und das Ansatzstück 11 bilden eine starre integrale Einheit, die ungeschlitzt und nicht aufklappbar ist. Auf einem Sokkel 18 am vorderen Ende des Ansatzstückes 11 sitzt klemmend das rückwärtige Ende einer Schutzhülse 19, welche die Punktionsnadel bzw. das Kanülenrohr umgibt und von der Punktionsnadel abgenommen werden kann.

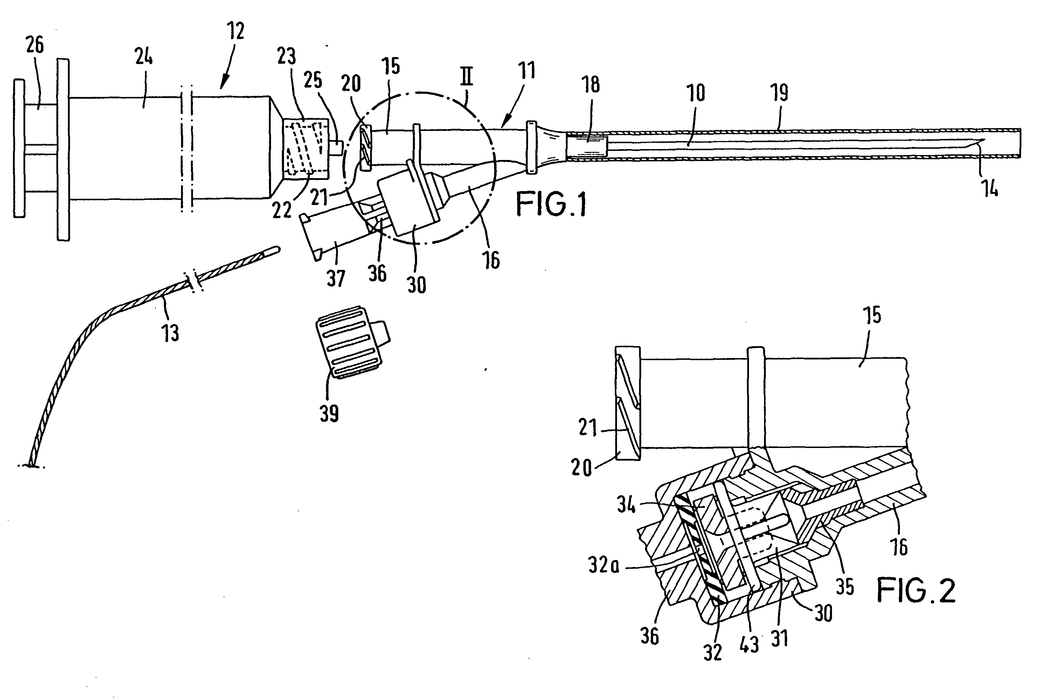

Am rückwärtigen Ende des Ansatzes 15 ist ein Verbindungsstück 20 für den Anschluß der Spritze 12 angeformt. Das Verbindungsstück 20 weist radiale Nocken 21 auf, die in Gewindegänge 22 im Innern des Halses 23 des Spritzenzylinders 24 eingreift, während der Spritzenkonus 25 abdichtend in den Kanal des Ansatzes 15 eindringt. Der Ansatz 15 bildet mit dem Verbindungsstück 20 den Spritzenhalter zum unmittelbaren (schlauchlosen) Ansetzen der Spritze 12. Der Spritzenzylinder 24 enthält einen Kolben, von dem in Fig. 1 nur die Kolbenstange 26 sichtbar ist. Durch Zurückziehen der Kolbenstange 26 kann nach erfolgter Venenpunktion Blut in die Spritze 12 aspiriert werden. Während des Punktionsvorganges kann die starr mit dem Ansatzstück 11 verbundene Spritze 12 als Handgriff zum Führen der Punktionsnadel benutzt werden.

Der seitlich abzweigende Ansatz 16 enthält in einer Kappe 30 ein Schlitzventil 31 und eine Lochscheibe 32, welche zusammen die Ventilvorrichtung bilden. Das Schlitzventil 31 ist in Fig. 5 dargestellt. Es weist einen Flansch 43 auf, an den sich ein becherförmiger Körper 44 anschließt. In der bogenförmigen Stirnwand 45 des Körpers 44 ist ein Schlitz 46 vorgesehen. Verstärkungsrippen 47,48 stehen nach entgegengesetzten Seiten von dem Körper 44 ab. Diese Verstärkungsrippen erstrecken sich auch über die Stirnwand 45 und sie enden zu beiden Seiten des Schlitzes 46. Zwischen der Lochscheibe 32 und dem Schlitzventil 31 ist ein trichterförmiges Drahtführungsteil 34 angeordnet, das mit einem Flansch zwischen die Flansche der Lochscheibe und des Schlitzventils eingespannt ist und in den Hohlraum des becherförmigen Schlitzventils 31 hineinragt. Ein weiteres trichterförmiges Drahtführungsteil 35 ist in Einschubrichtung des Führungsdrahtes 13 hinter dem Schlitzventil 31 angeordnet. Dieses Drahtführungsteil 35 ragt in den Kanal des Ansatzes 16 hinein.

Die Kappe 30 ist mit einem axialen Hals 36 versehen, der mit einem Einführtrichter 37 verbunden ist. Am Ende des Einführtrichters 37 befinden sich Verbindungselemente 38 zum Befestigen einer abnehmbaren Verschlußkappe 39, welche das Ende des Ansatzes 16 abdichtend verschließt.

Beim Einführen des Führungsdrahtes gelangt das vordere Ende des Führungsdrahtes zunächst in den Einführtrichter 37, wo es axial zentriert wird. Dann passiert der Führungsdraht 13 das Mittelloch 32a der Lochscheibe 32, dessen Durchmesser geringfügig kleiner ist als derjenige des Führungsdrahtes, so daß das Loch 32a aufgeweitet wird. Danach wird das vordere Ende des Führungsdrahtes 13 von dem Einführtrichter des Drahtführungsteils 34 wiederum zentriert, bevor es durch des Schlitz 46 des Schlitzventils 31 hindurchgeht. Das Schlitzventil 31 besteht aus einem einstückigen Elastomerkörper, dessen Schlitz 46 durch die Rippen 47,48 in Schließrichtung vorgespannt ist. Nach dem Passieren des Schlitzes 46 wird das Ende des Führungsdrahtes von dem zweiten Drahtführungsteil 35 wiederum zentriert. Der Führungsdraht dringt dann weiter durch den Kanal des Ansatzes 16 hindurch in das Innere der Punktionskanüle 10 vor. Das Schlitzventil 31 und die Lochscheibe 32 umschließen den Führungsdraht so eng, daß kein Blut entlang des Führungsdrahts aus dem Ansatz 16 austreten kann. Nach dem Herausziehen des Führungsdrahtes schließt das Schlitzventil 31 selbsttätig. Zusätzlich kann der Ansatz 16 dann noch mit der Kappe 39 verschlossen werden.

Das Verlegen eines (nicht dargestellten) Katheters mit der als Katheter-Einführungsvorrichtung dienenden Punktionskanüle geschieht wie folgt: das Blutgefäß wird mit der Punktionsnadel 10 punktiert, während die Spritze 12 auf dem rückwärtigen Ende des Ansatzes 15 sitzt. Der Punktionserfolg kann dadurch festgestellt werden, daß beim Zurückziehen der Kolbenstange 26 Blut in die Spritze eintritt. Blut, das hierbei in den Ansatz 16 gelangt, wird durch das Schlitzventil 31 zurückgehalten.

Wenn der Punktionserfolg festgestellt wurde, wird die Kappe 39 von dem Ansatz 16 abgenommen. Durch den Einführtrichter 37 wird der Führungsdraht 13 eingeführt, der eine flexible weiche Spitze haben kann. Der Führungsdraht durchdringt das Loch 32a der Lochscheibe 32 und anschließend den Schlitz 36 des Schlitzventils 31, wonach er jeweils durch die Drahtführungsteile 34 und 35 zentriert wird. Der Führungsdraht 13 tritt an der Spitze 14 aus der Punktionsnadel 10 aus, um weiter in die Vene hinein vorgeschoben zu werden. Nachdem der Führungsdraht auf diese Weise verlegt ist, wird die Punktionskanüle aus der Vene herausgezogen, während der Führungsdraht 13 festgehalten wird und in der Vene verbleibt. Beim Herausziehen der Punktionskanüle gleitet der Ansatz 16 über den Führungsdraht 13 hinweg. Nachdem der Führungsdraht verlegt ist, wird ein Katheter auf den Führungsdraht aufgeschoben und in die Vene hinein vorgeschoben. Danach wird der Führungsdraht aus dem Katheter herausgezogen.

## Patentansprüche

1. Venenpunktionskanüle für die Katheterverlegung nach der Seldinger-Technik, mit einer hohlen Punktionsnadel (10), einem am rückwärtigen Ende der Punktionsnadel (10) starr befestigten Y-förmigen Ansatzstück (11), das einen sich koaxial von der Punktionsnadel nach hinten erstreckenden, koaxial zur Punktionsnadel (10) verlaufenden ersten Ansatz (15) und einen hiervon seitlich schräg nach hinten abzweigenden zweiten Ansatz (16) aufweist, und einem Führungsdraht (13), wobei der zweite Ansatz einen Durchlaß für den Führungsdraht (13) aufweist und eine selbstschließende Abdichtvorrichtung enthält, und der erste Ansatz (15) als Spritzenhalter zum schlauchlosen Befestigen einer Spritze (12) unmittelbar am Ansatzstück (11) ausgebildet ist,
**dadurch gekennzeichnet,**
**daß** die selbstschließende Abdichtvorrichtung eine Ventilvorrichtung (31,32) ist, die durch Einschieben des Führungsdrahtes (13) öffnet und dabei den Führungsdraht abdichtend umschließt, daß die Ventilvorrichtung aus einem Schlitzventil (31) und einer elastischen Lochscheibe (32) besteht, die hintereinander in dem zweiten Ansatz (16) angeordnet sind, wobei der Lochdurchmesser der Lochscheibe (32) so eng ist, daß er von dem Führungsdraht (13) aufgeweitet wird, und dass zwischen der Lochscheibe (32) und dem Schlitzventil (31) ein trichterförmiges Drahtführungsteil (34) angeordnet ist.

2. Venenpunktionskanüle nach Anspruch 1, **dadurch gekennzeichnet, daß** der zweite Ansatz (16) unter einem Winkel von weniger als 35° zu dem ersten Ansatz (15) verläuft.

3. Venenpunktionskanüle nach Anspruch 1 oder 2, **dadurch gekennzeichnet, daß** der zweite Ansatz (16) in Einschubrichtung des Führungsdrahtes (13) vor der Ventilvorrichtung (31,32) einen Einführtrichter (37) für den Führungsdraht (13) enthält.

4. Venenpunktionskanüle nach einem der Ansprüche 1-3, **dadurch gekennzeichnet, daß** das Drahtführungsteil (34) in einen Hohlraum des becherförmigen Schlitzventils (31) hineinragt.

5. Venenpunktionskanüle nach einem der Ansprüche 1-4, **dadurch gekennzeichnet, daß** in Einschubrichtung des Führungsdrahtes (13) hinter dem Schlitzventil (31) ein weiteres trichterförmiges Drahtführungsteil (35) in dem Ansatz (16) angeordnet ist.

## Claims

1. A venipuncture cannula for placing catheters according to the Seldinger technique, comprising a hollow puncturing needle (10), a Y -shaped hub member (11) rigidly mounted at the rear end of the puncturing needle (10), the hub member having a first hub (15) extending rearward from the puncturing needle and coaxial with the puncturing needle (10) and a second hub (16) laterally branching obliquely rearward therefrom and a guide wire (13), the second hub having a passage for the guide wire (13) and a self-closing sealing device, and the first hub (15) being designed as a syringe holder for tubelessly mounting a syringe (12) immediately to the hub member (11),
**characterized in**
**that** the self-closing sealing device is a valve device (31, 32) opening upon insertion of the guide wire (13), while enclosing the guide wire in sealing manner, that the valve device comprises a slot valve (31) and an elastic disk (32) with a hole successively arranged in the second hub (16), the diameter of the hole of the disk (32) being so small that it is widened by the guide wire (13), and that a funnel-shaped wire guiding member (34) is located between the disk (32) with a hole and the slot valve (31).

2. The venipuncture cannula of claim 1, **characterized in that** the second hub (16) extends under an angle of less than 35° to the first hub (15).

3. The venipuncture cannula of claim 1 or 2, **characterized in that** the second hub (16) comprises an insertion funnel (37) for the guide wire (13) located upstream of the valve device (31, 32) seen in the direction of insertion of the guide wire (13).

4. The venipuncture cannula of one of claims 1-3, **characterized in that** the wire guiding member (34) protrudes into a cavity of the cup-shaped slot valve (31).

5. The venipuncture cannula of one of claims 1-4, **characterized in that** another funnel-shaped wire guiding member (35) is located in the hub (16) downstream of the slot valve (31) seen in the direction of insertion of the guide wire (13).

## Revendications

1. Canule de ponction de veine pour la pose d'un cathéter selon la technique de Seldinger, comportant une aiguille de ponction creuse (10), un embout (11) en forme de Y, qui est fixé rigidement à l'extrémité, dirigée vers l'arrière, de l'aiguille de ponction (10) et qui présente une première embouchure (15) s'étendant coaxialement vers l'arrière depuis l'aiguille de ponction, dans une position coaxiale à l'aiguille de ponction (10), et une seconde embouchure (16) formée en dérivation, obliquement vers l'arrière, sur le côté de la première, ainsi qu'un fil de guidage (13), la seconde embouchure présentant un passage pour le fil de guidage (13) et renfermant un dispositif d'étanchéité autoserrant, tandis que la première embouchure (15) est réalisée sous forme d'une monture de fixation de seringue, pour permettre de fixer une seringue (12) directement à l'embout (11), sans tuyau souple,
**caractérisée en ce que**
le dispositif d'étanchéité autoserrant est un dispositif formant clapet (31, 32), qui s'ouvre par insertion du fil de guidage (13) et enserre alors le fil de guidage en réalisant une étanchéité, **en ce que** le dispositif formant clapet est constitué d'un clapet à fente (31) et d'un disque à trou (32) élastique, qui sont disposés l'un à la suite de l'autre dans la seconde embouchure (16), le diamètre du trou du disque à trou (32) étant à ce point étroit qu'il est élargi par le fil de guidage (13), et **en ce qu'**entre le disque à trou (32) et le clapet à fente (31), est disposé un élément de guidage de fil (34) en forme d'entonnoir.

2. Canule de ponction de veine selon la revendication 1,
**caractérisée en ce que** la seconde embouchure (16) s'étend sous un angle de moins de 35° par rapport à la première embouchure (15).

3. Canule de ponction de veine selon la revendication 1 ou 2,
**caractérisée en ce que** la seconde embouchure (16) comporte, en amont du dispositif formant clapet (31, 32) suivant la direction d'insertion du fil de guidage (13), un entonnoir d'introduction (37) pour le fil de guidage (13).

4. Canule de ponction de veine selon l'une des revendications 1 à 3, **caractérisée en ce que** l'élément de guidage de fil (34) pénètre dans une cavité du clapet à fente (31) en forme de coupelle.

5. Canule de ponction de veine selon l'une des revendications 1 à 4, **caractérisée en ce qu'**en aval du clapet à fente (31) suivant la direction d'insertion du fil de guidage (13), est disposé, dans l'embouchure (16), un élément supplémentaire de guidage de fil (35) en forme d'entonnoir.
